# EUROPEAN PATENT APPLICATION

(11) **EP 4 817 598 A1**
(43) Date of publication of application: **07.10.2026**
(21) Application number: 24896683.0
(22) Date of filing: 29.11.2024
(51) Int. Cl.: A61K 9/08, A61K 39/395, A61K 47/26, A61P 35/00

(54) **STABLE PHARMACEUTICAL COMPOSITION OF MIXED ANTIBODIES**

(30) Priority: 01.12.2023 CN 202311642330; 21.11.2024 CN 202411675791
(71) Applicant: QILU PHARMACEUTICAL CO., LTD., Jinan, Shandong 250100 (CN)
(72) Inventor: ZHOU, Bingbing, Jinan, Shandong 250100 (CN); ZHANG, Le, Jinan, Shandong 250100 (CN); CONG, Riyuan, Jinan, Shandong 250100 (CN); AN, Zhenming, Jinan, Shandong 250100 (CN); LIU, Jun, Jinan, Shandong 250100 (CN); SUN, Lixia, Jinan, Shandong 250100 (CN); YANG, Chen, Jinan, Shandong 250100 (CN); ZHANG, Daoxing, Jinan, Shandong 250100 (CN); YU, Yue, Jinan, Shandong 250100 (CN); YE, Hongyan, Jinan, Shandong 250100 (CN); LI, Daoyuan, Jinan, Shandong 250100 (CN); WANG, Qingmin, Jinan, Shandong 250100 (CN); SUN, Bo, Jinan, Shandong 250100 (CN)
(74) Representative: ABG Intellectual Property Law, S.L.
(86) International application number: PCT/CN2024/135436
(87) International publication number: WO 2025/113598

(57) **Abstract**

Provided is a pharmaceutical composition containing anti-CTLA-4 and anti-PD-1 mixed antibodies, which has an excellent effect in terms of the stability, the safety, and the therapeutic efficacy, thereby effectively solving the problems of poor stability and high toxicity of a pharmaceutical composition containing anti-CTLA-4 and anti-PD-1 mixed antibodies.

## Description

This application claims priority to Chinese Patent Application No. CN2023116423306, entitled "STABLE PHARMACEUTICAL COMPOSITION OF MIXED ANTIBODIES" filed with the China National Intellectual Property Administration on December 1, 2023, and Chinese Patent Application No. CN2024116757918, entitled "STABLE PHARMACEUTICAL COMPOSITION OF MIXED ANTIBODIES" filed with the China National Intellectual Property Administration on November 21, 2024, the entire contents of which are incorporated herein by reference.

### TECHNICAL FIELD

The present disclosure relates to a stable pharmaceutical composition comprising an antibody mixture of an anti-CTLA-4 antibody and an anti-PD-1 antibody and use thereof, and pertains to the field of biotechnology.

### BACKGROUND

Programmed death receptor-1 (PD1 or PD-1) is a protein receptor of 288 amino acids expressed on activated T and B cells, natural killer cells, and monocytes. Its binding to a PD-1 ligand, PD-L1 or PD-L2, activates the PD-1 inhibitory signal, thereby inhibiting T-cell mediated immune responses against tumor cells. To combat this inhibition of the antitumor immune responses mediated by the PD-1 pathway, multiple companies have developed monoclonal antibodies that bind to human PD-1 and block the interaction between PD-1 and its ligands. Immune checkpoint therapy targeting the programmed death receptor 1 (PD-1) axis has led to breakthrough improvements in the clinical response to a variety of human cancers.

Cytotoxic T lymphocyte-associated protein 4 (CTLA4 or CTLA-4), is a member of the immunoglobulin superfamily, and consists of an extracellular V region, a transmembrane region, and a cytoplasmic region. CTLA-4 is homologous to the costimulatory molecule receptor CD28 on the surface of T cells, and both compete for binding to their ligands B7-1 (CD80) and B7-2 (CD86), which are mainly expressed on the surface of antigen-presenting cells. CTLA-4 is generally expressed on the surface of regulatory T cells (Tregs) and conventional T cells in an activated state. Upon binding to B7 molecules, CTLA-4 inhibits T cell activation, participates in negative regulation of immune responses, acts as an immune checkpoint and downregulates immune responses, and therefore, CTLA-4 plays a very important role in immune regulation.

ZPML265 is a pharmaceutical formulation of mixed antibodies comprised of a recombinant humanized IgG1 monoclonal antibody targeting human CTLA-4 and a recombinant humanized IgG4 monoclonal antibody targeting human PD-1, and the two different antibodies are produced by cells from a single host. The mixed antibodies can specifically bind to both of CTLA-4 and PD-1, thereby blocking the two immune checkpoint signal pathways of CTLA-4 and B7-1/B7-2, and PD-1 and PD-L1, abrogating the inhibitory effects of the two pathways on T lymphocytes, restoring the functional activity and anti-tumor immune response of T lymphocytes, and further enabling the body to achieve the purpose of fighting and killing tumors.

Compared with the traditional monoclonal antibodies, mixed antibodies offer favorable safety and tolerability, ease of administration, and remarkable therapeutic efficacy, with pharmacological effects significantly superior to those of single monoclonal antibodies. Meanwhile, compared with bispecific antibodies, mixed antibodies for combinatory use have shorter development cycle and if a higher concentration of antibody preparation can be developed, there can be shortened injection duration, reduced injection amount, increased patient compliance, reduced administration frequency of the antibodies, and improved production and storage efficiency. However, antibody drugs become unstable due to their large molecular weights, complex structures, easy degradation, polymerization, undesirable chemical modifications, and the like. In particular, mixed antibodies inherently exist as a mixture of two antibodies at low concentrations, and if high-concentration formulations are developed, the problem is that higher antibody/protein concentrations make it significantly more difficult to maintain proper activity and delivery parameters of the formulations. At present, there is an urgent need to develop stable pharmaceutical compositions at low and high concentrations suitable for the production and administration of mixed antibodies, and capable of maintaining stability and exerting a better therapeutic effect during storage and subsequent use.

### SUMMARY OF THE INVENTION

It is an object of the present disclosure to provide a pharmaceutical composition of mixed antibodies which is suitable for transport and storage, and has long-term high stability.

The present disclosure provides an aqueous pharmaceutical composition comprising an antibody mixture of an anti-CTLA-4 antibody and an anti-PD-1 antibody, wherein the pharmaceutical composition comprises the antibody mixture, a buffer, a stabilizer, and a surfactant.

In some embodiments, the heavy chain of the anti-CTLA-4 antibody has the amino acid sequence set forth in SEQ ID NO:1, the light chain of the anti-CTLA-4 antibody has the amino acid sequence set forth in SEQ ID NO:2, the heavy chain of the anti-PD-1 antibody has the amino acid sequence set forth in SEQ ID NO:3, and the light chain of the anti-PD-1 antibody has the amino acid sequence set forth in SEQ ID NO:4.

In some embodiments, the molar ratio of the anti-CTLA-4 antibody to the anti-PD-1 antibody in the antibody mixture is about 1: 2.

In some embodiments, the total concentration of the antibody mixture in the pharmaceutical composition is about 20-200 mg/ml.

In some embodiments, the antibody mixture is at a low concentration in the pharmaceutical composition, such as about 20-30 mg/ml, which includes, but is not limited to, about 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, or any value between any two of the above numbers.

In some embodiments, the antibody mixture is at a high concentration in the pharmaceutical composition, such as about 100-200 mg/ml, which includes, but is not limited to, about 100 mg/ml, 105 mg/ml, 110 mg/ml, 115 mg/ml, 120 mg/ml, 125 mg/ml, 130 mg/ml, 135 mg/ml, 140 mg/ml, 145 mg/ml, 150 mg/ml, 155 mg/ml, 160 mg/ml, 165 mg/ml, 170 mg/ml, 175 mg/ml, 180 mg/ml, 185 mg/ml, 190 mg/ml, 195 mg/ml, 200 mg/ml, or any value between any two of the above numbers.

In some embodiments, the buffer is selected from the group consisting of acetic acid-sodium hydroxide, histidine-hydrochloric acid, and histidine-acetic acid, and is at a concentration of about 5-50 mM, preferably about 10-20 mM, more preferably about 10 mM, 11 mM, 12 mM, 13 mM, 14 mM, 15 mM, 16 mM, 17 mM, 18 mM, 19 mM, 20 mM, or any value between any two of the above numbers.

In some embodiments, the stabilizer is selected from the group consisting of sucrose and trehalose, and is at a concentration of about 50-100 mg/ml, preferably about 60-100 mg/ml, more preferably about 60 mg/ml, 65 mg/ml, 70 mg/ml, 75 mg/ml, 80 mg/ml, 85 mg/ml, 90 mg/ml, 95 mg/ml, 100 mg/ml, or any value between any two of the above numbers.

In some embodiments, the surfactant is selected from the group consisting of polysorbate 20, polysorbate 80, and poloxamer 188, and is at a concentration of about 0.1-2 mg/ml, preferably about 0.1-1 mg/ml, more preferably about 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, or any value between any two of the above numbers.

In some embodiments, the pharmaceutical composition has a pH ranging from about 4.0-6.0, preferably about 4.7-5.6, and more preferably about 5.0-5.4.

In some embodiments, the pharmaceutical composition further comprises an antioxidant and/or a dispersant.

In some embodiments, the antioxidant is selected from methionine at a concentration of about 0-50 mM, preferably about 5-50 mM, and more preferably about 10 mM.

In some embodiments, the dispersant is selected from hyaluronidases at a concentration of about 0-4,000 IU/ml, preferably about 500-4,000 IU/ml, and more preferably about 2,000 IU/ml.

In some embodiments, the pharmaceutical composition may optionally further comprise arginine hydrochloride at a concentration of about 0-150 mM, preferably about 20-150 mM, more preferably about 100 mM.

The aqueous pharmaceutical composition at a low concentration described in the present disclosure comprises about 20-30 mg/ml of the antibody mixture, about 50-100 mg/ml of sucrose or about 50-100 mg/ml of trehalose, about 0.1-2 mg/ml of polysorbate 80 or about 0.1-2 mg/ml of polysorbate 20 or about 0.1-2 mg/ml of poloxamer 188, about 5-50 mM of acetic acid-sodium hydroxide or about 5-50 mM of histidine-hydrochloric acid or about 5-50 mM of histidine-acetic acid, wherein the aqueous pharmaceutical composition has a pH of about 4.0-6.0.

Further preferably, the aqueous pharmaceutical composition at a low concentration described in the present disclosure comprises about 20-30 mg/ml of the antibody mixture, about 80-100 mg/ml of sucrose or about 80-100 mg/ml of trehalose, about 0.1-0.5 mg/ml of polysorbate 80 or about 0.1-0.5 mg/ml of polysorbate 20 or about 0.1-0.5 mg/ml of poloxamer 188, about 10-50 mM of acetic acid-sodium hydroxide or about 10-50 mM of histidine-hydrochloric acid or about 10-50 mM of histidine-acetic acid, wherein the aqueous pharmaceutical composition has a pH of about 4.7-5.3.

The aqueous pharmaceutical composition at a low concentration described in the present disclosure comprises a component selected from the group consisting of:
Aqueous pharmaceutical composition A: about 20 mg/ml of the antibody mixture, about 50 mg/ml of sucrose, about 0.1 mg/ml of polysorbate 20, about 5 mM of acetic acid-sodium hydroxide, a pH of about 4.0;
Aqueous pharmaceutical composition B: about 20 mg/ml of the antibody mixture, about 100 mg/ml of sucrose, about 2 mg/ml of polysorbate 20, about 50 mM of acetic acid-sodium hydroxide, a pH of about 5.3;
Aqueous pharmaceutical composition C: about 25 mg/ml of the antibody mixture, about 60 mg/ml of sucrose, about 0.3 mg/ml of polysorbate 20, about 10 mM of acetic acid-sodium hydroxide, a pH of about 5.8;
Aqueous pharmaceutical composition D: about 25 mg/ml of the antibody mixture, about 70 mg/ml of sucrose, about 0.5 mg/ml of polysorbate 20, about 20 mM of acetic acid-sodium hydroxide, a pH of about 5.2;
Aqueous pharmaceutical composition E: about 25 mg/ml of the antibody mixture, about 90mg/ml of sucrose, about 0.2 mg/ml of polysorbate 20, about 10 mM of acetic acid-sodium hydroxide, a pH of about 5.0;
Aqueous pharmaceutical composition F: about 25 mg/ml of the antibody mixture, about 100 mg/ml of sucrose, about 1 mg/ml of polysorbate 20, about 30 mM of acetic acid-sodium hydroxide, a pH of about 6.0;
Aqueous pharmaceutical composition G: about 30 mg/ml of the antibody mixture, about 80 mg/ml of sucrose, about 0.6 mg/ml of polysorbate 20, about 20 mM of acetic acid-sodium hydroxide, a pH of about 4.7; and
Aqueous pharmaceutical composition H: about 30 mg/ml of the antibody mixture, about 100 mg/ml of sucrose, about 0.4 mg/ml of polysorbate 20, about 40 mM of acetic acid-sodium hydroxide, a pH of about 5.6.

The aqueous pharmaceutical composition at a high concentration described in the present disclosure comprises about 100-200 mg/ml of the antibody mixture, about 50-100 mg/ml of sucrose or about 50-100 mg/ml of trehalose, about 0.1-2 mg/ml of polysorbate 80 or about 0.1-2 mg/ml of polysorbate 20 or about 0.1-2 mg/ml of poloxamer 188, about 5-50 mM of acetic acid-sodium hydroxide or about 5-50 mM of histidine-hydrochloric acid or about 5-50 mM of histidine-acetic acid, wherein the aqueous pharmaceutical composition has a pH of about 4.0-6.0, and optionally, the aqueous pharmaceutical composition further comprises about 0-50 mM of methionine, and/or about 0-4000 IU/ml of hyaluronidase, and/or about 0-150 mM of arginine hydrochloride.

Further preferably, the aqueous pharmaceutical composition at a high concentration described in the present disclosure comprises about 100-200 mg/ml of the antibody mixture, about 50-100 mg/ml of sucrose or about 50-100 mg/ml of trehalose, about 0.1-2 mg/ml of polysorbate 80 or about 0.1-2 mg/ml of polysorbate 20 or about 0.1-2 mg/ml of poloxamer 188, about 5-50 mM of acetic acid-sodium hydroxide or about 5-50 mM of histidine-hydrochloric acid or about 5-50 mM of histidine-acetic acid, about 5-50 mM of methionine, about 500-4000 IU/ml of hyaluronidase, wherein the aqueous pharmaceutical composition has a pH of about 4.0-6.0, and optionally, the aqueous pharmaceutical composition further comprises about 20-150 mM of arginine hydrochloride.

Still further preferably, the aqueous pharmaceutical composition at a high concentration described in the present disclosure comprises about 100-200 mg/ml of the antibody mixture, about 60-90 mg/ml of sucrose or about 60-90 mg/ml of trehalose, about 0.2-1 mg/ml of polysorbate 80 or about 0.2-1 mg/ml of polysorbate 20 or about 0.2-1 mg/ml of poloxamer 188, about 10-20 mM of acetic acid-sodium hydroxide or about 10-20 mM of histidine-hydrochloric acid or about 10-20 mM of histidine-acetic acid, about 10 mM of methionine, about 2000 IU/ml of hyaluronidase, wherein the aqueous pharmaceutical composition has a pH of about 5.0-5.8, and optionally, the aqueous pharmaceutical composition further comprises about 100 mM of arginine hydrochloride.

The aqueous pharmaceutical composition at a high concentration described in the present disclosure comprises a component selected from the group consisting of:
Aqueous pharmaceutical composition I: about 100 mg/ml of the antibody mixture, about 50 mg/ml of sucrose, about 0.2 mg/ml polysorbate 20, about 10 mM of histidine-hydrochloric acid, a pH of about 5.0;
Aqueous pharmaceutical composition J: about 100 mg/ml of the antibody mixture, about 82 mg/ml of sucrose, about 0.8 mg/ml of polysorbate 20, about 15 mM of histidine-hydrochloric acid, a pH of about 5.4;
Aqueous pharmaceutical composition K: about 100 mg/ml of the antibody mixture, about 90 mg/ml of sucrose, about 1.5 mg/ml polysorbate 20, about 40 mM of histidine-hydrochloric acid, a pH of about 5.6;
Aqueous pharmaceutical composition L: about 120 mg/ml of the antibody mixture, about 80 mg/ml of sucrose, about 0.4 mg/ml polysorbate 20, about 13 mM of histidine-hydrochloric acid, a pH of about 5.0;
Aqueous pharmaceutical composition M: about 120 mg/ml of the antibody mixture, about 70 mg/ml of sucrose, about 0.6 mg/ml of polysorbate 20, about 10 mM of histidine-hydrochloric acid, about 10 mM of methionine, a pH of about 5.6;
Aqueous pharmaceutical composition N: about 120 mg/ml of the antibody mixture, about 63 mg/ml of sucrose, about 0.5 mg/ml of polysorbate 20, about 18 mM of histidine-hydrochloric acid, about 10 mM of methionine, a pH of about 5.4;
Aqueous pharmaceutical composition O: about 120 mg/ml of the antibody mixture, about 90 mg/ml of sucrose, about 1 mg/ml of polysorbate 20, about 20 mM of histidine-hydrochloric acid, about 10 mM of methionine, a pH of about 5.0;
Aqueous pharmaceutical composition P: about 150 mg/ml of the antibody mixture, about 100 mg/ml of sucrose, about 2 mg/ml of polysorbate 20, about 25 mM of histidine-hydrochloric acid, about 10 mM of methionine, a pH of about 5.8;
Aqueous pharmaceutical composition Q: about 150 mg/ml of the antibody mixture, about 60 mg/ml of sucrose, about 0.5 mg/ml of polysorbate 20, about 18 mM of histidine-hydrochloric acid, about 10 mM of methionine, a pH of about 5.0;
Aqueous pharmaceutical composition R: about 150 mg/ml of the antibody mixture, about 76mg/ml of sucrose, about 0.8 mg/ml of polysorbate 20, about 30 mM of histidine-hydrochloric acid, about 10 mM of methionine, a pH of about 5.4;
Aqueous pharmaceutical composition S: about 200 mg/ml of the antibody mixture, about 90 mg/ml of sucrose, about 0.7 mg/ml of polysorbate 20, about 50 mM of histidine-hydrochloric acid, about 10 mM of methionine, a pH of about 5.2;
Aqueous pharmaceutical composition T: about 200 mg/ml of the antibody mixture, about 70 mg/ml of sucrose, about 1 mg/ml of polysorbate 20, about 15 mM of histidine-hydrochloric acid, about 10 mM of methionine, a pH of about 5.4;
Aqueous pharmaceutical composition U: about 200 mg/ml of the antibody mixture, about 82 mg/ml of sucrose, about 0.5 mg/ml of polysorbate 20, about 10 mM of histidine-hydrochloric acid, about 10 mM of methionine, about 2000 IU/ml hyaluronidase, a pH of about 5.4;
Aqueous pharmaceutical composition V: about 150 mg/ml of the antibody mixture, about 76 mg/ml of sucrose, about 0.8 mg/ml of polysorbate 20, about 18 mM of histidine-hydrochloric acid, about 10 mM of methionine, about 2000 IU/ml hyaluronidase, a pH of about 5.4;
Aqueous pharmaceutical composition W: about 150 mg/ml of the antibody mixture, about 70 mg/ml of sucrose, about 0.6 mg/ml of polysorbate 20, about 15 mM of histidine-hydrochloric acid, about 10 mM of methionine, about 2000 IU/ml hyaluronidase, a pH of about 5.2;
Aqueous pharmaceutical composition X: about 120 mg/ml of the antibody mixture, about 63 mg/ml of sucrose, about 0.5 mg/ml of polysorbate 20, about 13 mM of histidine-hydrochloric acid, about 10 mM of methionine, about 2000 IU/ml hyaluronidase, pH of about 5.4;
Aqueous pharmaceutical composition Y: about 120 mg/ml of the antibody mixture, about 70 mg/ml of sucrose, about 0.8 mg/ml of polysorbate 20, about 25 mM of histidine-hydrochloric acid, about 10 mM of methionine, about 2000 IU/ml hyaluronidase, a pH of about 5.2; and
Aqueous pharmaceutical composition Z: about 100 mg/ml of the antibody mixture, about 60 mg/ml of sucrose, about 0.6 mg/ml of polysorbate 20, about 20 mM of histidine-hydrochloric acid, about 10 mM of methionine, about 2000 IU/ml hyaluronidase, a pH of about 5.2.

The present disclosure also provides a lyophilized formulation which is obtained by freeze-drying the aqueous pharmaceutical composition described in the present disclosure, or which, upon reconstitution, yields the aqueous pharmaceutical composition described in the present disclosure.

The present disclosure also provides use of the aqueous pharmaceutical composition or lyophilized preparation in the manufacture of a medicament for the treatment of a tumor selected from the group consisting of esophageal squamous cell carcinoma, melanoma, cervical cancer, small cell lung cancer, non-small cell lung cancer, colorectal cancer, liver cancer, and renal cancer.

### BRIEF DESCRIPTION OF THE DRAWINGS

FIG. 1 is a graph showing changes in tumor volume after treatment with ZPML265, an anti-PD-1 antibody, an anti-CTLA-4 antibody and IgG.
Figure 2 shows changes in tumor volume in different groups of mice.
Figure 3 shows tumor weights in different groups of mice.
Figure 4 shows changes in body weight in different groups of mice.

### DETAILED DESCRIPTION

### Terminology

All publications, patents, and patent applications mentioned in this specification are incorporated herein by reference to the same extent as if each individual publication, patent, or patent application had been specifically and individually indicated to be incorporated by reference.

Before the disclosure is described in detail below, it is to be understood that the disclosure is not limited to the specific methodologies, protocols, and reagents described herein, as these may vary. It is also to be understood that the terminology used herein is for the purpose of describing particular embodiments only and is not intended to limit the scope of the present disclosure. Unless defined otherwise, all technical and scientific terms used herein have the same meaning as commonly understood by one of ordinary skill in the art to which this disclosure belongs.

Certain embodiments disclosed herein include numerical ranges, and certain aspects of the disclosure can be described by means of ranges. Unless otherwise indicated, it is to be understood that the numerical ranges or descriptions in the form of ranges are for purposes of brevity and convenience only, and should not to be considered as a strict limitation on the scope of the present disclosure. Accordingly, the descriptions in the form of ranges should be considered to specifically disclose all possible sub-ranges and all possible specific numerical points within that range, as if such sub-ranges and numerical points had been expressly described herein. The above principles apply equally regardless of the width of the numerical values. When a range description is employed, the range includes the endpoints of the range.

By means of "about" includes and describes a value or parameter itself. For example, "about x" includes and describes "x" itself. As used herein, the term "about", when used in conjunction with a measured value or for modifying a value, unit, constant or series of values, refers to variations within ±20%, or in some cases within ±10%, or in some cases within ±5%, or in some cases within ±1%, or in some cases within ±0.1% of the stated value, in addition to the value or parameter itself.

Three-letter and single-letter codes for amino acids as used herein are as described in J. Chem, 243, p3558(1968).

The term "antibody" as used herein typically refers to a Y-type tetrameric protein comprising two heavy (H) polypeptide chains and two light (L) polypeptide chains held together by covalent disulfide bonds and non-covalent interactions. Native IgG antibodies have such a structure. Each light chain comprises a variable domain (VL) and a constant domain (CL). Each heavy chain comprises a variable domain (VH) and a constant domain (CH).

Five major classes of antibodies are known in the art: IgA, IgD, IgE, IgG and IgM, and the corresponding heavy chain constant domains are referred to as alpha, delta, epsilon, gamma and mu, respectively. IgG and IgA can be further divided into different subclasses, for example, IgG can be divided into IgG1, IgG2, IgG3, and IgG4, and IgA can be divided into IgA1 and IgA2. The light chain of an antibody from any vertebrate species may be assigned to one of two distinct types, called kappa and lambda, based on the amino acid sequence of its constant domain.

As used herein, the types of "antibodies" in a broad sense can include, for example, polyclonal antibodies, monoclonal antibodies, chimeric antibodies, humanized and primatized antibodies, CDR-grafted antibodies, human antibodies (including recombinantly produced human antibodies), recombinantly produced antibodies, intracellular antibodies, multispecific antibodies, bispecific antibodies, monovalent antibodies, multivalent antibodies, anti-idiotypic antibodies, synthetic antibodies (including muteins and variants thereof), and the like.

The term "monoclonal antibody" (or "mAb") refers to an antibody produced by a single cell clone that is substantially homogeneous, and is directed against only a certain specific antigenic epitope. Monoclonal antibodies can be prepared using a variety of techniques known in the art, including hybridoma techniques, recombinant techniques, phage display techniques, transgenic animals, synthetic techniques, or a combination of the above techniques.

The term "antibody mixture" refers to a mixture comprising a limited number of major antibody species (optionally no more than two, three, four, five, six, seven, eight, nine or ten) produced by host cells (optionally cells from a single host cell line) that have been transfected with DNAs encoding at least two different antibodies with different binding specificities (optionally full-length primate IgG antibodies). In some embodiments, DNAs encoding at least two different heavy chains (HCs) and at least two different light chains (LCs) can be introduced into a same host cell, and for example, the host cell can be transfected with DNAs encoding at least two but no more than four different antibodies with different binding specificities. In some embodiments, the sequences of all transfected DNAs encoding HCs and LCs can be mutated, thereby changing the amino acid sequences of the antibodies, such that non-homologous HC/LC pairing is disadvantageous, and homologous HC/LC pairing is highly advantageous. When two different HCs are introduced into host cells, one or both of the two different HCs may optionally be altered so as to disfavor the formation of heterodimers. In some embodiments, only one heavy chain is altered to prevent heterodimer formation. In some embodiments, where DNAs encoding only two different antibodies are introduced into host cells, only one of the antibodies encoded by the DNAs comprises one or more partner-directed alterations such that homologous HC/LC pairing is facilitated, whereas the other antibody does not comprise such alterations. In some embodiments, only two major antibody species are produced by host cells, each HC primarily paired with its homologous LC, and most antibodies are tetramers containing two heavy chains with the same amino acid sequence and two light chains with the same amino acid sequence (see PCT/US2017/030676).

The terms "pharmaceutical formulation", "formulation" and "formulation prescription" are used interchangeably herein to denote an article of manufacture that is present in a form that allows the effective presence of a pharmaceutical active ingredient, and that does not contain other components that are toxic to a subject to whom the formulation is to be administered.

The term "pharmaceutical composition" refers to a formulation or a combination of formulations comprising one, two or more active ingredients that allows the active ingredients contained therein to exist in a biologically active and effective form and that does not contain additional ingredients that have unacceptable toxicity to a subject to which the formulation is administered. When a "pharmaceutical composition" exists as a combination of separate formulations containing two or more different active ingredients, it may be administered simultaneously, sequentially, separately or at intervals, the purpose of which is to exert the biological activities of various active ingredients and jointly treat diseases.

"ZPML265" refers to an antibody mixture comprising an anti-CTLA-4 antibody and an anti-PD-1 antibody, in which a heavy chain of the anti-CTLA-4 antibody is as set forth in SEQ ID NO:1, a light chain of the anti-CTLA-4 antibody is as set forth in SEQ ID NO:2, a heavy chain of the anti-PD-1 antibody is as set forth in SEQ ID NO:3, and a light chain of the anti-PD-1 antibody is as set forth in SEQ ID NO:4. "ZPML265 Injection" refers to a low-concentration formulation of ZPML265 for administration to a subject for the treatment of a disease. "ZPML265H Injection" refers to a high-concentration formulation of ZPML265 for administration to a subject for the treatment of a disease.

The term "high-concentration" formulation refers to a formulation comprising the active pharmaceutical ingredient (API) at a concentration of about 100 mg/ml-200 mg/ml.

The term "effective amount" refers to a dosage of a pharmaceutical formulation comprising the active ingredient of the present disclosure that, upon administration to a patient in a single dose or multiple doses, produces the desired effect in the patient being treated. An effective amount can be readily determined by the attending physician as a person skilled in the art by considering a variety of factors such as ethnic differences; weight, age, and health status; the particular disease involved; severity of the disease; response of an individual patient; specific antibodies administered; mode of administration; bioavailability profile of the administered formulation; the selected dosing regimen and the use of any concomitant therapy.

The term "pharmaceutically acceptable excipients" refers to those substances/reagents that can be reasonably administered to a subject to provide an effective amount of the active pharmaceutical ingredient in a stable formulated form. Suitable pharmaceutically acceptable excipients are well known in the art and include, but are not limited to, buffers, stabilizers, surfactants, and the like.

The term "buffer" refers to a pharmaceutically acceptable excipient that stabilizes the pH of a pharmaceutical formulation. Suitable buffers are well known in the art and can be found in the literature.

The term "stabilizer" refers to a pharmaceutically acceptable excipient for protecting active pharmaceutical ingredients and/or formulations from chemical and/or physical degradation during production, storage and application. The term "surfactant" refers to a pharmaceutically acceptable excipient for protecting protein formulations from mechanical stress such as agitation and shearing.

The term "antioxidant" refers to a compound that prevents oxidation of an active ingredient under storage conditions.

The term "dispersant" refers to a compound for increasing the permeability of an active substance into tissues (e.g., skin) and aiding in the effective diffusion of the active substance, such as hyaluronidase, also known as HAase, which can hydrolyze hyaluronic acid, thereby reducing the viscosity of hyaluronic acid in the extracellular matrix, and allowing the pharmaceutical formulation to disperse more easily in tissues.

The term "lyophilized formulation" refers to a composition obtained or obtainable by a freeze-drying process of a liquid formulation. Preferably, it is a solid composition with a water content of less than 5%, preferably less than 3%.

The term "treatment" refers to a clinical intervention in an attempt to alter a person or to treat a disease caused by cells, either prophylactically or in a clinically pathological process. Therapeutic effects include, but are not limited to, preventing the occurrence or recurrence of a disease, alleviating symptoms, reducing the direct or indirect pathological consequences of any disease, preventing metastasis, slowing the rate of disease progression, improving or alleviating the condition, and alleviating or improving the prognosis.

The term "individual", "subject" or "patient" refers to any animal, such as mammals or marsupials. Individuals of the present disclosure include, but are not limited to, humans, non-human primates (e.g., cynomolgus monkeys or rhesus monkeys or other types of macaques), mice, pigs, horses, donkeys, cattle, sheep, rats, and any kind of poultry.

The term "disease" or "condition" or "disorder" or the like refers to any change or disorder that damages or interferes with the normal function of cells, tissues or organs. For example, the "disease" includes, but is not limited to, tumors, pathogen infections, autoimmune diseases, T cell dysfunction diseases, or immunotolerance deficiency (e.g., transplant rejection).

The term "tumor" refers to a disease characterized by pathological proliferation of cells or tissues, and their subsequent migration or invasion into other tissues or organs. Tumor growth is generally uncontrolled and progressive and does not induce or inhibit normal cell proliferation. Tumors include "cancer," which generally refers to all malignancies.

The term "combination" refers to a treatment regimen that provides at least two or more different therapies to achieve a specified therapeutic effect, the therapies may be either physical, such as radiation therapy, or chemical, such as administration of a drug to a subject, and the drug also includes drugs for combinatory use. "Drugs for combinatory use" refers to a combination of two or more pharmaceutical formulations each containing an active ingredient, which need to be used in combination when administered to a subject. The active ingredients can be mixed together to form a single administration unit, or can be used separately as administration units, respectively: upon administration, different pharmaceutical formulations can be administered substantially simultaneously, simultaneously or sequentially.

Recurrence-free survival (RFS) is defined as the period from randomization to the first recorded disease recurrence (local, regional, or distant), or death from any cause (whichever occurs first).

Overall survival (OS) refers to the period from the initiation of study treatment to death from any cause in a subject. For patients who are still under follow-up as of the analysis cut-off date, the analysis cut-off date shall be taken as the censoring time, and for subjects who are lost to follow-up, the date of the last contact with them shall be taken as the censoring time.

Progression-free survival (PFS) refers to the period from the initiation of study treatment to the first disease progression evaluated by imageology or death of any cause (whichever occurs first) in a subject.

Objective remission rate (ORR) is defined as the best confirmed ORR during the study, including cases of complete response (CR) and partial response (PR), and according to RECISTvl.1, when PR or CR occurs for the first time, an additional imageological examination of the lesions needs to be performed for confirmation at ≥4 weeks.

Disease control rate (DCR) refers to the percentage of cases with an optimal therapeutic effect evaluated as "CR+PR+SD".

Complete remission (CR): All target lesions disappear and the short diameter of all pathological lymph nodes (including target nodules and non-target nodules) must be reduced to <10mm.

Partial remission (PR): The sum of target lesion diameters is reduced by at least 30% compared with the baseline levels.

Progressive disease (PD): The sum of the diameters is relatively increased by at least 20% with reference to the minimum value of the sum of the diameters of all target lesions measured throughout the experimental study (reference to the baseline value if the baseline measurement is minimum): in addition, the absolute value of the sum of the diameters must be increased by at least 5 mm (the appearance of one or more new lesions is also considered progressive disease).

Stable disease (SD): The extent of reduction in target lesions does not reach PR levels and the extent of increase in target lesions does not reach PD levels, which is between the above two levels, and the minimum of the sum of diameters can be used as the reference in the study.

Duration of remission (DOR) is defined as the period from the first assessment of a tumor as CR or PR (whichever is recorded first) to the first assessment of PD or death.

Treatment-emergent adverse event (TEAE) refers to an adverse event occurring during treatment.

Treatment-related adverse event (TRAE) refers to an adverse event occurring during treatment that is associated with a study drug.

"Serious adverse event (SAE)" refers to any adverse medical event (AE) that occurs after a subject receives a study drug, including death, life-threatening condition, permanent or severe disability or loss of function, the need for hospitalization or prolongation of hospitalize time, and congenital abnormality or birth defect.

Dose-limiting toxicity (DLT) is defined as Grade ≥3 non-hematological toxicity or Grade ≥4 hematological toxicity occurring within 21 days (1 cycle) after the first administration.

Maximum tolerated dose (MTD) is defined as the highest dose level of a drug at which no more than 1 of 6 subjects experiences the DLT during the 21-day (1 cycle) treatment period after the first administration.

### Experimental Example 1: ELISA Method

An IL-2 ELISA kit was diluted with PSB, and then a plate was coated with 4 µg/mL capture antibody at 50 µL/well. The plate was sealed and incubated overnight. The coating solution was aspirated, and 100 µL of SmartBlock (#NC0104327, Fisher Scientific) was added to each well, and the plate was incubated for 1 hour. The blocking solution was aspirated, and the supernatant sample diluted 15-fold with ELISA buffer was added to the plate along with the IL-2 standard provided in the ELISA kit. After incubation for 2 hours, the plate was washed using a plate washer, in which the program was set to 3 cycles of wash and aspiration. The IL-2 detection antibody in the ELISA kit was added to the ELISA buffer to prepare a solution at a concentration of 0.100 µg/mL, 50 µL of the solution was added to each well, and the plate was incubated for 1 hour. The plate was washed again using the plate washer. Streptavidin-labeled HRP (#21140, Pierce) was diluted 1:5000 in the ELISA buffer, and 50 µL of the diluted HRP was added to each well, followed by incubation for 30 minutes. The plate was washed using the plate washer. Then, 50 µL of TMB substrate (#tmbw-1000-01, SurModics) was added to each well, and color development was allowed to proceed for 4 minutes at room temperature. The reaction was stopped by adding 50 µL of 1 N H₂SO₄ to each well. The plate was then read at 450-570 nm. Raw data were transferred to graphing software (GraphPad Prism) in which the data was converted into a sigmoidal curve fitting graph. Subsequent analysis (linear regression calculation) was performed in GraphPad Prism to generate EC₅₀ values.

### Experimental Example 2: Determination of Protein Content

The protein content was determined using a Lunatic microspectrometer. A blank subtraction was performed with purified water, the ultraviolet absorption at 280 nm was corrected by light scattering at 330 nm, and the protein concentration was calculated according to Equation 1, wherein the extinction coefficient ε was 1.64 (L/g·cm⁻¹). $Protein content = \frac{A \left(280\right) - A \left(330\right)}{ε}$ Equation 1

### Experimental Example 3: Size-Exclusion Chromatography (SEC-HPLC)

Aggregates, monomers and fragments were quantified using size exclusion chromatography. The method was run on a Waters e2695-2489 HPLC system using Waters Xbridge BEH SEC 200Å, 7.8×300mm column. The mobile phase was 100 mM sodium phosphate, and 150 mM sodium chloride, pH 6.8. The sample was diluted to 1 mg/ml with the mobile phase and the injection volume was 25 µL. Isocratic elution was carried out at a flow rate of 0.5 mL/min for 30 min and the detection wavelength was 215 nm. Integration was performed using Empower 3 software, and the percentage content of each component was calculated according to the area normalization method.

### Experimental Example 4: Imaged Capillary Isoelectric Focusing (iCIEF)

In imaged capillary isoelectric focusing, carrier ampholytes establish a uniform pH gradient increasing from low to high from the anode to the cathode within the capillary under an electric field, and protein charge variants with different charges are focused and separated in the pH gradient of the ampholytes according to their differences in isoelectric point. The focusing process was monitored in real time at 280 nm wavelength. An FC-coated fused-silica capillary with an internal diameter of 100 µm and an effective separation length of 5 cm was used. When a sample was treated, Cytiva Pharmalyte 3-10 at a final concentration of 1%, Cytiva Pharmalyte 8-10.5 at a final concentration of 0.5%, HPMC at a final concentration of 0.35%, and urea at a final concentration of 1 M were respectively added, and the final concentration of the sample was 0.5 mg/ml. The voltage and time of focusing separation were 1.5kV-1min and 3kV-6min. The electropherogram were integrated using Empower 3 software. The contents of the acidic region, main peak and basic region were calculated as percent peak area, and the pI value of the target peak was calculated using the pI value of the Marker.

### Experimental Example 5: Detection of Hyaluronidase Activity

The activity of hyaluronidase was detected by turbidimetry. Hyaluronidase can enzymatically hydrolyze hyaluronic acid substrate, and excess hyaluronic acid solution can form a stable colloidal solution with acidified serum. A standard curve was plotted by the measured corresponding absorbance against different concentrations of standard, based on which the activity of hyaluronidase can be calculated. A low-protein-binding 96-well sample plate containing a test sample and standard, and a 96-well substrate plate containing a hyaluronic acid working solution were incubated at 37°C respectively. The test sample and the standard were taken from the sample plate, and were added to the substrate plate for reaction, and the serum working solution was added to the substrate plate to terminate the reaction. The absorbance was measured by reading the plate at 640 nm, and the activity of the test sample was calculated according to the standard curve.

### Examples

Specific examples are set forth below to illustrate the disclosure, and it is to be understood that these examples are merely illustrative of the disclosure and are not intended to limit the scope of the disclosure.

### Example 1: Preparation of Antibody Mixture ZPML265

An antibody mixture produced by a single host cell line was prepared (see PCT/US2017/030676). The antibody mixture comprises two active ingredients, i.e., a recombinant humanized IgG1 monoclonal antibody targeting human CTLA4 and a recombinant humanized IgG4 monoclonal antibody targeting human PD1. The antibody mixture can specifically bind to both of human CTLA4 and PD1. After both of the antibodies were expressed simultaneously by a single host cell line, the antibodies were collected, purified, and combined with a pharmaceutically acceptable carrier to form the single antibody mixture ZPML265. The amino acid sequences of each of the anti-CTLA4 antibody and the anti-PD1 antibody were shown in Table 1 below. The molar ratio of the anti-PD-1 antibody to the anti-CTLA-4 antibody in ZPML265 was 2:1.

**Table 1: Amino acid sequence of each component of antibody mixture ZPML265**

| Name | SEQ ID NO: |
|---|---|
| Heavy chain HC of anti-CTLA4 antibody | 1 |
| Light chain LC of anti-CTLA4 antibody | 2 |
| Heavy chain HC of anti-PD1 antibody | 3 |
| Light chain LC of anti-PD1 antibody | 4 |

### Example 2: Study on Types of Buffers in Low-Concentration Formulation

In order to optimize the types of buffers in the ZPML265 formulation, 10 mM of acetic acid-sodium hydroxide, 10 mM of histidine-hydrochloric acid and 10 mM of histidine-acetic acid were respectively selected as the buffers to evaluate the stability of the formulations. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulation solutions were placed at 40°C for 1 month (1M), 2-8°C for 1 month, and 25°C for 1 month to evaluate sample stability. The information for the different formulations (R1, R2 and R3) is shown in Table 2, and the stability results for the different formulations are shown in Table 3.

**Table 2: Formulation information**

| Formulation | R1 | R2 | R3 |
|---|---|---|---|
| ZPML265 | 25 mg/ml | 25 mg/ml | 25 mg/ml |
| Sucrose | 90 mg/ml | 90 mg/ml | 90 mg/ml |
| Acetic acid-sodium hydroxide | 10mM | / | / |
| Histidine-hydrochloric acid | / | 10mM | / |
| Histidine-acetic acid | / | / | 10mM |
| Polysorbate 20 | 0.2 mg/ml | 0.2 mg/ml | 0.2 mg/ml |
| pH | 5.0 | 5.0 | 5.0 |

**Table 3: Stability data**

| Sample name | SEC-HPLC (%) | | |
|---|---|---|---|
| | Aggregate | Main peak | Fragment |
| R1 0h | 0.25 | 99.7 | 0.09 |
| R2 0h | 0.23 | 99.7 | 0.09 |
| R3 0h | 0.23 | 99.7 | 0.09 |
| R1 40°C 1M | 0.58 | 98.6 | 0.85 |
| R2 40°C 1M | 0.51 | 97.8 | 1.7 |
| R3 40°C 1M | 0.63 | 98.4 | 0.94 |
| R1 25°C 1M | 0.26 | 99.6 | 0.19 |
| R2 25°C 1M | 0.22 | 99.5 | 0.32 |
| R3 25°C 1M | 0.27 | 99.5 | 0.20 |
| R1 2-8°C 1M | 0.24 | 99.7 | 0.11 |
| R2 2-8°C 1M | 0.21 | 99.7 | 0.13 |
| R3 2-8°C 1M | 0.22 | 99.7 | 0.12 |

The above results show that as evidenced by the stability evaluation for one month, there was no significant difference between formulations R1 and R3, and acetic acid-sodium hydroxide and histidine-acetic acid can be used as buffers.

### Example 3: Study on Concentrations of Buffers in Low-Concentration Formulation

In order to optimize the concentrations of buffers in the ZPML265 formulation, acetic acid-sodium hydroxide at concentrations of 10 mM, 20 mM, and 50 mM was selected as the buffer to evaluate the stability of the formulations. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed at 40°C for 3 weeks (3W) to evaluate sample stability. The information for the different formulations is shown in Table 4, and the stability results for the different formulations are shown in Table 5.

**Table 4: Formulation information**

| | R1 | R2 | R3 |
|---|---|---|---|
| Antibody concentration | 30 mg/ml | 30 mg/ml | 30 mg/ml |
| Sucrose | 90 mg/ml | 90 mg/ml | 90 mg/ml |
| Acetic acid-sodium hydroxide | 10mM | 20mM | 50mM |
| pH | 5.0 | 5.0 | 5.0 |

**Table 5: Stability data**

| Sample name | SEC-HPLC (%) | | |
|---|---|---|---|
| | Aggregate | Main peak | Fragment |
| R1 0h | 2.6 | 97.2 | 0.2 |
| R2 0h | 2.6 | 97.3 | 0.2 |
| R3 0h | 3.0 | 96.9 | 0.2 |
| R1 40°C 3W | 5.5 | 93.3 | 1.2 |
| R2 40°C 3W | 5.6 | 93.2 | 1.2 |
| R3 40°C 3W | 6.8 | 92.1 | 1.1 |

The above results show that, the aggregate content in the formulation containing 50 mM buffer was higher than that in the formulations containing 10 mM and 20 mM buffers after storage at 40°C for 3 weeks, and therefore, the formulation with 10 mM or 20 mM buffer was preferable.

### Example 4: Study on Surfactants in Low-Concentration Formulation

In order to optimize the types and concentrations of surfactants in the ZPML265 formulation, polysorbate 20 and polysorbate 80 with concentrations of 0.2 mg/ml and 0.5 mg/ml were selected as surfactants respectively, to evaluate the stability of the formulations. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed at 40°C for 3 weeks (3W) and shaken for 24h (150 rpm, 25°C) to evaluate sample stability. The information for the different formulations is shown in Table 6, and the stability results for the different formulations are shown in Table 7.

**Table 6: Formulation information**

| | R1 | R2 | R3 | R4 | R5 |
|---|---|---|---|---|---|
| Antibody concentration | 30 mg/ml | 30 mg/ml | 30 mg/ml | 30 mg/ml | 30 mg/ml |
| Sucrose | 90 mg/ml | 90 mg/ml | 90 mg/ml | 90 mg/ml | 90 mg/ml |
| Acetic acid-sodium hydroxide | 10mM | 10mM | 10mM | 10mM | 10mM |
| Polysorbate | 0.2 mg/ml PS20 | 0.5 mg/ml PS20 | 0.2 mg/ml PS80 | 0.5 mg/ml PS80 | / |
| pH | 5.0 | 5.0 | 5.0 | 5.0 | 5.0 |

**Table 7: Stability data**

| Sample name | SEC-HPLC (%) | | | MFI (number of particles of ≥5µm) |
|---|---|---|---|---|
| | Aggregate | Main peak | Fragment | |
| R1 0h | 0.7 | 99.2 | 0.1 | 1064 |
| R2 0h | 0.7 | 99.2 | 0.1 | 1077 |
| R3 0h | 0.7 | 99.2 | 0.1 | 1762 |
| R4 0h | 0.7 | 99.2 | 0.1 | 951 |
| R5 0h | 0.7 | 99.2 | 0.1 | 7053 |
| R1, shaking for 24 h | 0.7 | 99.2 | 0.1 | 444 |
| R2, shaking for 24 h | 0.8 | 99.1 | 0.1 | 771 |
| R3, shaking for 24 h | 0.7 | 99.2 | 0.1 | 609 |
| R4, shaking for 24 h | 0.7 | 99.2 | 0.1 | 1790 |
| R5, shaking for 24 h | 0.7 | 99.2 | 0.1 | 20306 |
| R1 40°C 3W | 1.3 | 98.5 | 0.3 | 1721 |
| R2 40°C 3W | 1.8 | 97.9 | 0.4 | 1291 |
| R3 40°C 3W | 1.6 | 98.0 | 0.3 | 1943 |
| R4 40°C 3W | 1.6 | 98.0 | 0.4 | 282 |
| R5 40°C 3W | 1.5 | 98.2 | 0.3 | 18846 |

The above results show that as evidenced by the stability evaluation, the number of particles in the formulation without polysorbate was significantly greater than that in the formulations with polysorbate. There was no significant difference between the use pf polysorbate 20 and polysorbate 80. Purities of the main peaks of the formulations containing 0.2 mg/ml of polysorbate 20 were slightly higher. Therefore 0.2 mg/ml of polysorbate 20 was preferred as a surfactant.

### Example 5: DOE study on Low-Concentration Formulation

Nine formulations were designed for the DOE study, in which the protein concentration, polysorbate 20 content, sucrose content, and pH were taken as variables and the buffers were 10 mM of acetic acid-sodium hydroxide. The effects of interaction of polysorbate 20, pH, protein concentration, and sucrose content on formulation stability were evaluated. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed at 40°C for 1 month and 2-8°C for 3 months to evaluate sample stability. The information for the different formulations is shown in Table 8, and the stability results for the different formulations are shown in Table 9.

**Table 8: Formulation information**

| Formulation No. | pH | Protein content (mg/ml) | Polysorbate 20 content(mg/ml) | Sucrose content (mg/ml) |
|---|---|---|---|---|
| R1 | 4.7 | 20 | 0.1 | 80 |
| R2 | 5.3 | 30 | 0.1 | 80 |
| R3 | 4.7 | 30 | 0.1 | 100 |
| R4 | 5.0 | 25 | 0.2 | 90 |
| R5 | 4.7 | 20 | 0.3 | 100 |
| R6 | 5.3 | 30 | 0.3 | 100 |
| R7 | 5.3 | 20 | 0.3 | 80 |
| R8 | 5.3 | 20 | 0.1 | 100 |
| R9 | 4.7 | 30 | 0.3 | 80 |

**Table 9: Stability data**

| Sample name | SEC-HPLC (%) | | |
|---|---|---|---|
| | HMWS (%) | Monomer (%) | LMWS (%) |
| 0h R1 | 0.19 | 99.7 | 0.11 |
| 0h R2 | 0.2 | 99.7 | 0.11 |
| 0h R3 | 0.19 | 99.7 | 0.11 |
| 0h R4 | 0.2 | 99.7 | 0.11 |
| 0h R5 | 0.21 | 99.7 | 0.12 |
| 0h R6 | 0.22 | 99.7 | 0.11 |
| 0h R7 | 0.22 | 99.7 | 0.11 |
| 0h R8 | 0.2 | 99.7 | 0.11 |
| 0h R9 | 0.2 | 99.7 | 0.11 |
| 40°C 1M R1 | 0.22 | 98.7 | 1.1 |
| 40°C 1M R2 | 0.47 | 98.8 | 0.72 |
| 40°C 1M R3 | 0.29 | 98.6 | 1.1 |
| 40°C 1M R4 | 0.45 | 98.6 | 0.96 |
| 40°C 1M R5 | 0.28 | 98.6 | 1.2 |
| 40°C 1M R6 | 0.47 | 98.8 | 0.75 |
| 40°C 1M R7 | 0.50 | 98.7 | 0.77 |
| 40°C 1M R8 | 0.36 | 98.9 | 0.75 |
| 40°C 1M R9 | 0.29 | 98.5 | 1.2 |
| 2-8°C 3M R1 | 0.14 | 99.4 | 0.44 |
| 2-8°C 3M R2 | 0.19 | 99.4 | 0.43 |
| 2-8°C 3M R3 | 0.15 | 99.4 | 0.42 |
| 2-8°C 3M R4 | 0.16 | 99.4 | 0.43 |
| 2-8°C 3M R5 | 0.14 | 99.4 | 0.47 |
| 2-8°C 3M R6 | 0.19 | 99.4 | 0.46 |
| 2-8°C 3M R7 | 0.19 | 99.4 | 0.47 |
| 2-8°C 3M R8 | 0.16 | 99.4 | 0.44 |
| 2-8°C 3M R9 | 0.15 | 99.4 | 0.48 |

### Example 6: In Vivo Pharmacodynamic Studies of Anti-PD-1 Component, Anti-CTLA-4 Component and ZPML265 in Humanized NCG Mouse HCC827 Tumor Model

NCG immunodeficient mice were used as experimental mice, into which human tumor cells were firstly inoculated, followed by intravenous injection of human PBMCs to build a tumor-bearing mouse model with human immune system. ZPML265, anti-human PD-1 antibody (the anti-PD-1 component of ZPML265), and anti-human CTLA-4 antibody (the anti-CTLA-4 component of ZPML265) were then administered to the mice separately. The therapeutic efficacy of the anti-PD-1 antibody, anti-CTLA-4 antibody, and ZPML265 in the humanized NCG mouse HCC827 tumor model were studied, and whether ZPML265 had synergistic therapeutic efficacy was explored.

PBMCs were isolated from peripheral blood of healthy donors by density gradient centrifugation and resuspended with PBS to 1×10⁸ cells/mL for inoculation. After the density of HCC827 tumor cells was adjusted with PBS, 5×10⁶ cells/100 µL/mouse were inoculated subcutaneously into the right flank of NCG mice, and the day on which the tumor cells were inoculated was recorded as day 0. When the mean tumor volume reached 60-80 mm³ (approximately 5 days after tumor cell inoculation), 1×10⁷ PBMC/100 µL/mouse were injected intravenously, and drugs were administered intraperitoneally 1 hour after PBMC inoculation. Before dosing, the animals were randomly divided into 4 groups (5 mice/group) using StudyDirector^{™} software based on the tumor volume of the selected animals. The NCG mice were intraperitoneally (i.p.) administered the low-concentration formulation of ZPML265 (7.5 mg/kg), anti-PD-1 antibody (5 mg/kg), anti-CTLA-4 antibody (2.5 mg/kg), and human IgG1 (7.5 mg/kg). Dosing was performed twice a week for 3 consecutive weeks. The tumors were measured for long diameter and short diameter twice a week using a vernier caliper, and weighed, and the tumor volumes expressed as mm³ were calculated using the following formula: V = 0.5 × a × b², where a and b represent the long and short diameters of the tumor, respectively. Tumor growth inhibition (TGI) and tumor volume were used as biological indicators for pharmacodynamics (PD) in this study.

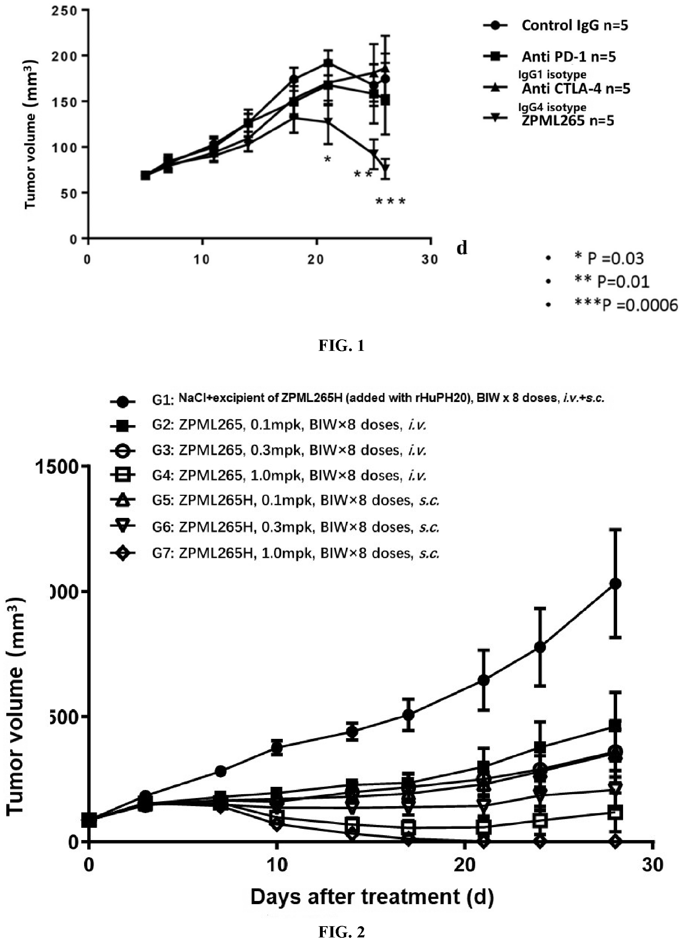

No abnormal clinical signs were observed in the animals during the experiment. As shown in FIG. 1, the low-concentration formulation of ZPML265 significantly inhibited tumor growth (TGI=93%, 7.5 mg/kg), whereas the anti-PD-1 antibody or anti-CTLA-4 antibody administered alone showed no significant therapeutic efficacy.

In summary, in the experiment of humanized NCG mouse HCC827 tumor model, the inhibitory effect of the low-concentration formulation of ZPML265 on HCC827 tumors was superior to that of the anti-PD-1 antibody or anti-CTLA-4 antibody alone, and ZPML265 had a synergistic therapeutic efficacy.

### Example 7:High-Concentration Formulation

To prepare a high-concentration formulation solution of ZPML265, ZPML265 was buffer exchanged using an intended excipient solution and concentrated by ultrafiltration to a desired antibody concentration. After the ultrafiltration concentration was completed, the excipient to be added were added to the antibody solution in the form of a stock solution, and finally the protein concentration was adjusted to a desired concentration of about 100 mg/ml, 120 mg/ml, 150 mg/ml, 200 mg/ml, and the like with a buffer. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed at 25°C for 2 months (2M) and 40°C for 1 month (1M) to evaluate sample stability. The information for the different formulations is shown in Table 10, and the stability results for the different formulations are shown in Table 11.

**Table 10: Formulation information**

| | R1 | R2 | R3 | R4 | R5 |
|---|---|---|---|---|---|
| Antibody concentration | 100 mg/ml | 150 mg/ml | 200 mg/ml | 100 mg/ml | 120 mg/ml |
| Sucrose | 60 mg/ml | 60 mg/ml | 60 mg/ml | 90 mg/ml | 90 mg/ml |
| Acetic acid-sodium hydroxide | 10mM | 10mM | 10mM | 10mM | 10mM |
| Polysorbate 20 | 0.5 mg/ml | 0.5 mg/ml | 0.5 mg/ml | 0.5 mg/ml | 0.5 mg/ml |
| Arginine hydrochloride | 100mM | 100mM | 100mM | 100mM | 100mM |
| Methionine | / | / | / | / | 10mM |
| pH | 5.5 | 5.5 | 5.5 | 5.5 | 5.0 |

**Table 11: Stability data**

| Sample name | SEC-HPLC (%) | | |
|---|---|---|---|
| | Aggregate | Main peak | Fragment |
| R1 0h | 0.8 | 99.1 | 0.11 |
| R2 0h | 0.93 | 99.0 | 0.10 |
| R3 0h | 0.89 | 99.0 | 0.11 |
| R4 0h | 0.75 | 99.1 | 0.11 |
| R5 0h | 0.59 | 99.3 | 0.13 |
| R1 40°C 1M | 2.9 | 94.4 | 2.7 |
| R2 40°C 1M | 3.2 | 94.2 | 2.6 |
| R3 40°C 1M | 3.5 | 93.9 | 2.6 |
| R4 40°C 1M | 2.3 | 95.0 | 2.7 |
| R5 40°C 1M | 2.0 | 93.9 | 4.14 |
| R1 25°C2M | 1.9 | 97.9 | 0.24 |
| R2 25°C2M | 2.0 | 97.7 | 0.25 |
| R3 25°C2M | 2.3 | 97.5 | 0.23 |
| R4 25°C2M | 1.5 | 98.3 | 0.24 |
| R5 25°C2M | 1.2 | 98.5 | 0.35 |

The above results show that as evidenced by the stability evaluation, the aggregate content increased with the increase of protein concentration after the protein concentration exceeded 120 mg/ml. Therefore, the protein concentration was preferably 120 mg/ml. There was no significant difference between formulations comprising different concentrations of sucrose as shown by comparisons between formulation 1 and formulation 4, and between formulation 2 and formulation 5. As evidenced by the stability evaluation at a high temperature of 40°C for 1 month (1M), the aggregate in the methionine-containing formulation was lower as shown by a comparison between formulation 4 and formulation 5. Therefore, 10 mM of methionine was preferred.

### Example 8: Surfactants in High-Concentration Formulation

In the high-concentration formulation, concentrations of the surfactant and arginine hydrochloride were further optimized. Polysorbate 20 at concentrations of 0.5 mg/ml and 1 mg/ml, poloxamer 188 at concentrations of 0.2 mg/ml and 0.5 mg/ml, and arginine hydrochloride at concentrations of 0 mg/ml and 100 mg/ml were selected to evaluate the stability of the formulation. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed under the following conditions to evaluate sample stability: three freeze-thaw cycles, shaking for 5 days at 25 °C, 40 °C for 1 month, 2-8 °C for 6 months, and 25 °C for 3 months. The information for the different formulations is shown in Table 12, the stability results for the different formulations are shown in Table 13, and the microparticle results for the different formulations are shown in Table 14.

**Table 12: Formulation information**

| | R1 | R2 | R3 | R4 | R5 |
|---|---|---|---|---|---|
| Antibody concentration | 120 mg/ml | 120 mg/ml | 120 mg/ml | 120 mg/ml | 120 mg/ml |
| Sucrose | 60 mg/ml | 60 mg/ml | 60 mg/ml | 60 mg/ml | 60 mg/ml |
| Acetic acid-sodium hydroxide | 10mM | 10mM | 10mM | 10mM | 10mM |
| Arginine hydrochloride | / | 100mM | 100mM | 100mM | 100mM |
| Polysorbate 20 | 0.5 mg/ml | 0.5 mg/ml | 1 mg/ml | / | / |
| Poloxamer 188 | / | / | / | 0.5 mg/ml | 0.2 mg/ml |
| Methionine | 10mM | 10mM | 10mM | 10mM | 10mM |
| pH | 5.0 | 5.0 | 5.0 | 5.0 | 5.0 |

**Table 13: Stability data**

| Conditions for evaluation | SEC-HPLC (%) | | |
|---|---|---|---|
| | Aggregate | Main peak | Fragment |
| R1 0h | 0.48 | 99.5 | / |
| R2 0h | 0.47 | 99.5 | / |
| R3 0h | 0.49 | 99.5 | / |
| R4 0h | 0.48 | 99.5 | / |
| R5 0h | 0.48 | 99.5 | / |
| R1, three freeze-thaw cycles | 0.81 | 99.2 | / |
| R2, three freeze-thaw cycles | 0.61 | 99.4 | / |
| R3, three freeze-thaw cycles | 0.7 | 99.3 | / |
| R4, three freeze-thaw cycles | 0.64 | 99.4 | / |
| R5, three freeze-thaw cycles | 0.63 | 99.4 | / |
| R1, shaking for 5 days | 0.89 | 99.1 | / |
| R2, shaking for 5 days | 0.91 | 99.1 | / |
| R3, shaking for 5 days | 0.88 | 99.1 | / |
| R4, shaking for 5 days | 0.79 | 99.2 | / |
| R5, shaking for 5 days | 0.79 | 99.2 | / |
| R1 40°C 1M | 2.8 | 96.7 | 0.56 |
| R2 40°C 1M | 2.7 | 96.7 | 0.64 |
| R3 40°C 1M | 2.6 | 96.7 | 0.68 |
| R4 40°C 1M | 2.3 | 97.0 | 0.72 |
| R5 40°C 1M | 2.8 | 96.6 | 0.65 |
| R1 25°C 3M | 2.2 | 97.4 | 0.32 |
| R2 25°C 3M | 1.9 | 97.8 | 0.34 |
| R3 25°C 3M | 2.1 | 97.6 | 0.34 |
| R4 25°C 3M | 1.9 | 97.8 | 0.32 |
| R5 25°C 3M | 1.9 | 97.8 | 0.33 |
| R1 2-8°C 6M | 1.4 | 97.8 | 0.84 |
| R2 2-8°C 6M | 1.3 | 97.8 | 0.85 |
| R3 2-8°C 6M | 1.4 | 97.7 | 0.86 |
| R4 2-8°C 6M | 1.4 | 97.8 | 0.86 |
| R5 2-8°C 6M | 1.3 | 97.9 | 0.8 |

**Table 14 Microparticle data**

| Microparticles of ≥5µm | | | | |
|---|---|---|---|---|
| | Silicone oil or bubble | Dense aggregates | Protein aggregates or protein fibers | Leachable from packaging materials |
| R1, shaking for 5 days | 209 | 74 | 53 | 7 |
| R2, shaking for 5 days | 436 | 101 | 7 | 0 |
| R3, shaking for 5 days | 317 | 108 | 19 | 10 |
| R4, shaking for 5 days | 83 | 35 | 269 | 10 |
| R5, shaking for 5 days | 17 | 26 | 480 | 14 |

The above results show that as evidenced by the stability evaluation, the presence or absence of arginine hydrochloride in the formulations has no significant effect on the purity of the main peak when comparing formulation 1 and formulation 2, Among formulation 2, formulation 3, formulation 4 and formulation 5, formulation 4 and formulation 5 had slightly more protein aggregates in microparticles of ≥5µm, while there was no significant difference between formulations with different concentrations of polysorbate 20 when comparing formulation 2 and formulation 3. Polysorbate 20 was preferred as a surfactant.

### Example 9: pH of High-Concentration Formulation

In order to improve subcutaneous absorption of the high-concentration formulation, hyaluronidase was introduced into the formulation, and the pH of the formulation was further optimized. The stability of the formulation was evaluated at pH 5.0, 5.2, 5.4, 5.6, and 5.8 with an enzymatic activity of 2000 IU/ml, respectively. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed at 40 °C for 1 month, 2-8 °C for 3 months, and 25 °C for 1 month to evaluate sample stability. The information for the different formulations is shown in Table 15, and the stability results for the different formulations are shown in Table 16.

**Table 15: Formulation information**

| | R1 | R2 | R3 | R4 | R5 |
|---|---|---|---|---|---|
| Antibody concentration | 120 mg/ml | 120 mg/ml | 120 mg/ml | 120 mg/ml | 120 mg/ml |
| Sucrose | 90 mg/ml | 90 mg/ml | 90 mg/ml | 90 mg/ml | 90 mg/ml |
| Acetic acid-sodium hydroxide | 10mM | 10mM | 10mM | 10mM | 10mM |
| Polysorbate 20 | 0.5 mg/ml | 0.5 mg/ml | 0.5 mg/ml | 0.5 mg/ml | 0.5 mg/ml |
| Methionine | 10mM | 10mM | 10mM | 10mM | 10mM |
| Hyaluronidase | 2000 U/ml | 2000 U/ml | 2000 U/ml | 2000 U/ml | 2000 U/ml |
| pH | 5.0 | 5.2 | 5.4 | 5.6 | 5.8 |

**Table 16: Stability data**

| Conditions for evaluation | Enzymatic activity (IU/ml) | SEC-HPLC (%) | | |
|---|---|---|---|---|
| | | Aggregate | Main peak | Fragment |
| R1 0h | 2182 | 0.23 | 99.7 | 0.11 |
| R2 0h | 2382 | 0.25 | 99.6 | 0.12 |
| R3 0h | 2300 | 0.28 | 99.6 | 0.12 |
| R4 0h | 2125 | 0.32 | 99.6 | 0.12 |
| R5 0h | 2238 | 0.28 | 99.6 | 0.11 |
| R1 25°C 1M | 2339 | 0.5 | 99.2 | 0.34 |
| R2 25°C 1M | 2439 | 0.55 | 99.2 | 0.22 |
| R3 25°C 1M | 2384 | 0.63 | 99.2 | 0.17 |
| R4 25°C 1M | 2521 | 0.63 | 99.2 | 0.18 |
| R5 25°C 1M | 2481 | 0.65 | 99.2 | 0.17 |
| R1 40°C 1M | / | 1.5 | 95.7 | 2.8 |
| R2 40°C 1M | / | 1.2 | 96.6 | 2.2 |
| R3 40°C 1M | / | 1.3 | 96.7 | 2.03 |
| R4 40°C 1M | / | 1.4 | 96.7 | 1.9 |
| R5 40°C 1M | / | 1.3 | 96.9 | 1.8 |
| R1 2-8°C 3M | 1887.0 | 0.36 | 99.4 | 0.24 |
| R2 2-8°C 3M | 2038.0 | 0.41 | 99.4 | 0.17 |
| R3 2-8°C 3M | 1932.0 | 0.47 | 99.4 | 0.14 |
| R4 2-8°C 3M | 2088.0 | 0.52 | 99.4 | 0.14 |
| R5 2-8°C 3M | 2150.0 | 0.5 | 99.4 | 0.14 |

The above results show that as evidenced by the stability evaluation, there was no significant difference in the purity of the SEC main peak at 2-8°C for 3 months, at 25°C for 1 month and at 40°C for 1 month, and the preferred pH was 5.4.

### Example 10: Buffers in High-Concentration Formulation

The buffer in the high-concentration formulation was further optimized, and 10 mM and 20 mM of acetic acid-sodium hydroxide, as well as 10 mM and 20 mM of histidine-hydrochloric acid were selected as buffers to evaluate the stability of the formulation. All prepared formulations were aseptically filtered through a 0.22 µm low-protein-binding filter, and filled into sterile 2 ml tubular vials under aseptic conditions and the vials were sealed with membrane-coated stoppers and aluminum-plastic combination caps. These formulations were placed at 40 °C for 1 month, 2-8 °C for 3 months, 25 °C for 3 months and 30°C for 3 months to evaluate sample stability. The information for the different formulations is shown in Table 17, and the stability results for the different formulations are shown in Table 18.

**Table 17: Formulation information**

| | R1 | R2 | R3 | R4 |
|---|---|---|---|---|
| Antibody concentration | 120 mg/ml | 120 mg/ml | 120 mg/ml | 120 mg/ml |
| Sucrose | 90 mg/ml | 90 mg/ml | 90 mg/ml | 90 mg/ml |
| Acetic acid-sodium hydroxide | 10mM | 20mM | / | / |
| Histidine-hydrochloric acid | / | / | 10mM | 20mM |
| Polysorbate 20 | 0.5 mg/ml | 0.5 mg/ml | 0.5 mg/ml | 0.5 mg/ml |
| Methionine | 10mM | 10mM | 10mM | 10mM |
| Hyaluronidase | 2000 IU/ml | 2000 IU/ml | 2000 IU/ml | 2000 IU/ml |
| pH | 5.4 | 5.4 | 5.4 | 5.4 |

**Table 18: Stability data**

| Conditions for evaluation | Enzymatic activity (IU/ml) | SEC-HPLC (%) | | |
|---|---|---|---|---|
| | | Aggregate | Main peak | Fragment |
| R1 0h | 2154 | 0.58 | 99.3 | 0.11 |
| R2 0h | 2095 | 0.57 | 99.3 | 0.12 |
| R3 0h | 2163 | 0.63 | 99.3 | 0.11 |
| R4 0h | 2118 | 0.5 | 99.4 | 0.12 |
| R1 40°C 1M | / | 2.4 | 95.6 | 2.0 |
| R2 40°C 1M | / | 2.2 | 95.7 | 2.1 |
| R3 40°C 1M | / | 2.0 | 95.8 | 2.2 |
| R4 40°C 1M | / | 1.8 | 96.0 | 2.2 |
| R1 2-8°C 3M | 1719 | 0.96 | 98.6 | 0.49 |
| R2 2-8°C 3M | 1705 | 0.93 | 98.6 | 0.48 |
| R3 2-8°C 3M | 1840 | 0.98 | 98.6 | 0.48 |
| R4 2-8°C 3M | 1810 | 0.84 | 98.7 | 0.48 |
| R1 25°C 3M | 1594 | 1.7 | 96.9 | 1.4 |
| R2 25°C3M | 1551 | 1.6 | 97.0 | 1.5 |
| R3 25°C 3M | 1799 | 1.5 | 97.1 | 1.4 |
| R4 25°C 3M | 1831 | 1.3 | 97.3 | 1.4 |
| R1 30°C 3M | 1360 | 2.2 | 96.1 | 1.7 |
| R2 30°C 3M | 1249 | 2.2 | 96.0 | 1.8 |
| R3 30°C 3M | 1820 | 1.9 | 96.4 | 1.8 |
| R4 30°C 3M | 1777 | 1.6 | 96.6 | 1.8 |

The above results show that the enzyme activities of formulation 3 and formulation 4 were high and the aggregate content in formulation 4 was low as evidenced by the stability evaluation at 30°C for 3 months. Therefore, 20 mM of histidine-hydrochloric acid was preferred as a buffer.

### Example 11: Study on Stability of Enzymatic Activities of High-Concentration Formulation

The stability of enzymatic activities of the high-concentration formulation was further evaluated. All prepared formulations were aseptically filtered through a 0.22 µm low-protein-binding filter, and filled into sterile 2 ml tubular vials under aseptic conditions, and the vials were sealed with membrane-coated stoppers and aluminum-plastic combination caps. These formulations were placed at 40 °C for 7 day, 2-8 °C for 3 months, 6 months and 9 months, 25 °C for 1 month, 3 months and 6 months to evaluate sample stability. The information for the different formulations is shown in Table 19, the stability results for the different formulations are shown in Table 20, and the enzymatic activity results for formulation R1 are shown in Table 21.

**Table 19: Formulation information**

| | R1 | R2 |
|---|---|---|
| Antibody concentration | 120 mg/ml | 120 mg/ml |
| Sucrose | 70 mg/ml | 70 mg/ml |
| Histidine-hydrochloric acid | 20mM | 20mM |
| Polysorbate 20 | 0.5 mg/ml | 0.5 mg/ml |
| Methionine | 10mM | 10mM |
| Hyaluronidase | 2000 IU/ml | / |
| pH | 5.4 | 5.4 |

**Table 20: Stability data**

| Conditions for evaluation | SEC-HPLC(%) | | |
|---|---|---|---|
| | Aggregate | Main peak | Fragment |
| R1 0h | 0.71 | 99.3 | 0.04 |
| R2 0h | 0.51 | 99.4 | 0.12 |
| R1 40°C 7D | 1.1 | 96.9 | 2.0 |
| R2 40°C 7D | 1.1 | 96.9 | 2.1 |
| R1 2-8°C 3M | 0.67 | 98.6 | 0.72 |
| R2 2-8°C 3M | 0.66 | 98.6 | 0.74 |
| R1 25°C 3M | 1.2 | 95.7 | 3.0 |
| R2 25°C 3M | 1.2 | 95.9 | 3.0 |
| R1 2-8°C 9M | 0.86 | 96.5 | 2.6 |
| R2 2-8°C 9M | 0.84 | 96.5 | 2.6 |

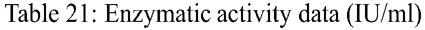

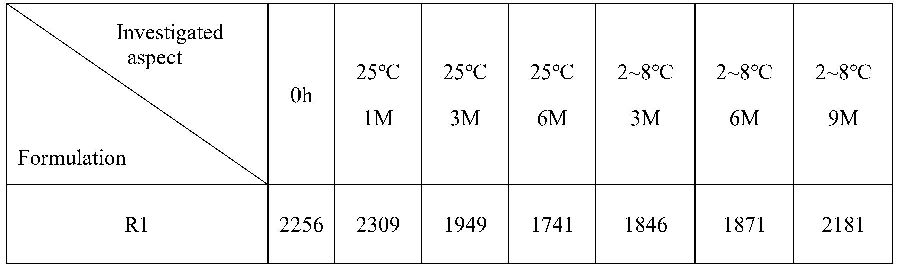

The above results show that as evidenced by the stability evaluation, there was no significant difference in purity between formulation 1 and formulation 2, indicating that hyaluronidase had no significant effect on stability. There was no significant change in enzymatic activity of formulation 1, indicating good stability of enzyme activity.

### Example 12: Evaluation of Anti-Tumor Effects of ZPML265H Injection and ZPML265 Injection in B6-hPD1hCTLA4 Mice Model Subcutaneously Transplanted with MC38-hPDL1

MC38-hPDL1 tumor cells were subcutaneously inoculated into the right flank of B6-hPD1/hCTLA4 humanized mice (from GemPharmatech LLC.) at 1×10⁶ cells/100 µl/mouse. When the average tumor volume reached 86.72 mm³, 56 mice were randomly divided into 7 groups (8 mice per group) according to their body weights and tumor volumes. The mice in group G1 as control were given NaCl+ excipient (already added with hyaluronidase rHuPH20) of ZPML265H injection. The mice in groups G2-G4 were given different dosages of ZPML265 injection (0.1 mg/kg for group G2, 0.3 mg/kg for group G3, and 1 mg/kg for group G4), respectively. The mice in groups G5-G7 were given different dosages of ZPML265H injection (0.1 mg/kg for Group G5, 0.3 mg/kg for Group G6, and 1 mg/kg for Group G7), respectively. The frequency of administration was twice a week for a total of 8 administrations. NaCl was administered via tail vein and the excipient (already added with hyaluronidase rHuPH20) of ZPML265H injection was administered subcutaneously to the mice in group G1. The mice in groups G2-G4 received administrations by tail vein, while the mice in groups G5-G7 received subcutaneous administrations. The day of grouping was defined as D0, the dosing period was from D0 to D28, with administrations on D0, D3, D7, D10, D14, D17, D21, and D24, and the experiment was terminated on D28. After initiation of dosing, tumor sizes were measured and body weights of the mice were weighed on D0, D3, D7, D10, D14, D17, D21, D24, and D28. The formula for calculating the tumor volume was: tumor volume (mm³) =0.5×long diameter of tumor×(short diameter of tumor)². At the end of the experiment, the animals were euthanized and analyzed for changes in tumor volume (TGITV), in tumor weight (TGITW) and in body weight. Results of the experiments were expressed as mean±standard error (Mean ± SEM). Data were analyzed using SPSS 24 and the independent sample T-test (T-Test) was used for comparison between two groups. P < 0.05 was considered statistically significant. Graphs were generated using Graphpad prism 9.

As shown in FIG. 2, at the end of the experiment, D28, values of tumor growth inhibition of tumor volume (TGITV) were 56.42%, 66.36% and 89.44% in the groups administered with ZPML265 at the dosages of 0.1, 0.3 and 1 mg/kg, respectively, while values of TGITV in the groups administered with ZPML265H at the dosages of 0.1, 0.3 and 1 mg/kg were 66.85%, 80.35% and 99.78%, respectively. Compared with NaCl+ excipient (already added with hyaluronidase rHuPH20) of ZPML265H injection in the control group, each of the groups administered with the test samples ZPML265 and ZPML265H had significantly differences in tumor volume, indicating that ZPML265 and ZPML265H at different dosages had a significant inhibitory effect on tumor volume in a dose-dependent manner.

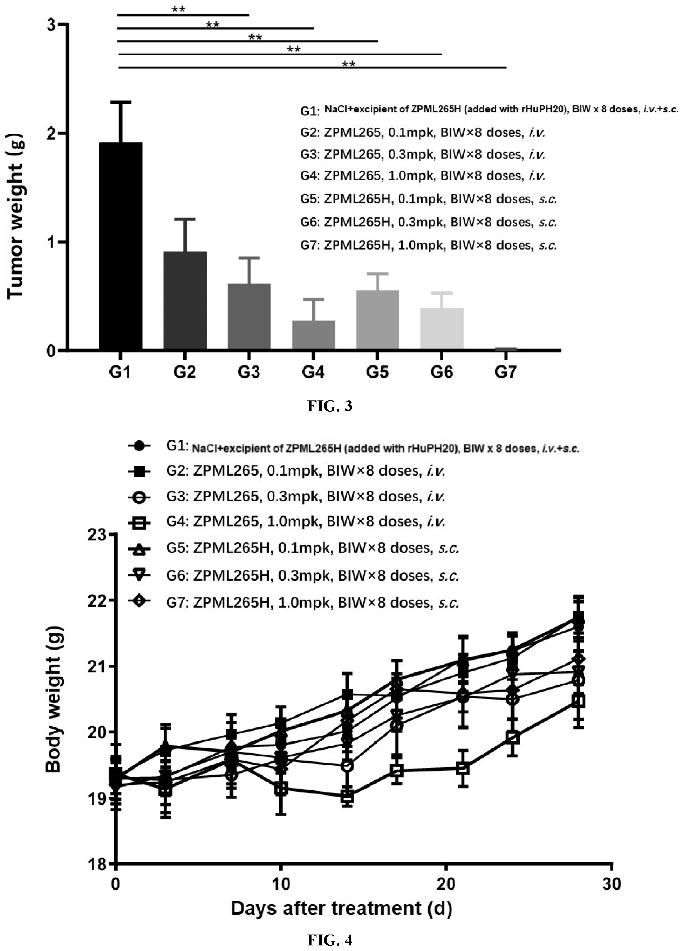

As shown in FIG. 3, the tumor weight results were consistent with the trend of the tumor volume results, values of tumor growth inhibition of tumor weight (TGITW) were 52.46%, 68.02% and 85.54% in the groups administered with ZPML265 at the dosages of 0.1, 0.3 and 1 mg/kg, respectively, while values of TGITW in the groups administered with ZPML265H at the dosages of 0.1, 0.3 and 1 mg/kg were 71.14%, 79.83% and 99.66%, respectively. Compared with NaCl+ excipient (already added with hyaluronidase rHuPH20) of ZPML265H injection in the control group, each of the groups administered with ZPML265 and ZPML265H had significantly reduced tumor weights in a dose-dependent manner.

The therapeutic efficacy of ZPML265H and ZPML265 injections were compared at the same dosage, and the results showed that there was no significant difference in tumor volume and tumor weight between the ZPML265H and ZPML265 groups at the same dosage. The above results indicated that the inhibitory effect of ZPML265H injection on tumors was comparable to that of ZPML265 injection at the same dosage.

As shown in FIG. 4, compared with NaCl+ excipient (already added with hyaluronidase rHuPH20) of NaCl+ZPML265H injection in the control group, there was no significant difference in body weight of the mice in each of the groups administered with ZPML265 and ZPML265H, except for a significant decrease in body weight of the animals in ZPML265 (at a dosage of 1 mg/kg), indicating that the safety of ZPML265H injection had a tendency to be superior to that of ZPML265 injection at the same dosage.

The results of the above study indicated that ZPML265 and ZPML265H injections at different dosages were capable of significantly inhibiting tumor growth in a dose-dependent manner. The therapeutical efficacy of ZPML265H injection was comparable to that of ZPML265 injection at the same dosage, and the safety of ZPML265H injection had a tendency to be superior to that of ZPML265 injection.

### Example 13: A Phase II Clinical Study to Evaluate the Safety and Therapeutical Efficacy of ZPML265 Injection in Combination with Paclitaxel-Cisplatin/Carboplatin in Patients with Recurrent or Metastatic Cervical Cancer Administration

In a phase II clinical study conducted in China, 30 patients with recurrent or stage IVB cervical cancer were enrolled, who were over 18 years old, had an Eastern Cooperative Oncology Group (ECOG) performance status score of 0 or 1, an expected survival of ≥ 3 months, and had the following functional levels of important organs prior to the first use of investigational product:
1) absolute neutrophil count ≥1.5×10⁹/L;
2) platelets≥100×10⁹/L;
3) hemoglobin ≥90 g/L;
4) serum albumin ≥30 g/L;
5) alanine aminotransferase (AST) and aspartate aminotransferase (ALT) ≤2.5× upper limit of normal (ULN) (≤5×ULN was permitted for patients with liver cancer or liver metastases);
6) total bilirubin ≤1.5×ULN (≤3×ULN was permitted for patients with Gilbert's syndrome);
7) serum creatinine ≤1.5×ULN, if the creatinine level of a patient was >1.5×ULN, creatinine clearance (CLcr) calculated by the Cockcroft-Gault equation must be≥50 mL/min;
8) left ventricular ejection fraction (LVEF)>50%;
9) proteinuria<2+(when urine protein of a patient was ≥2+, 24-hour urine protein should be quantified and the patient can be enrolled if the amount of the 24-hour urine protein was ≤1g).
10) international normalized ratio (INR) ≤1.5; and activated partial thromboplastin time (APTT) ≤1.5×ULN.

Dosing regimen. ZPML265 injection was administered by intravenous infusion at a dose of 5 mg/kg every three weeks.

The drugs used in this study were as follows. The following drugs were administered every 3 weeks, with each 21-day period (3 weeks) as 1 cycle. Paclitaxel and cisplatin/carboplatin were administered for a maximum of 6 cycles. After 6 cycles, subjects received ZPML265 injection to maintain therapy until disease progression, intolerable toxicity, initiation of other antitumor therapies, loss to follow-up, death, withdrawal from the study or the like occurred (whichever occurred first). The dosing window was ±3 days from the scheduled dosing date (calculated from the date after the first administration), and if the dosing window was exceeded, it was regarded as early or delayed administration, and the subsequent dosing date shall be recalculated according to the last actual dosing date.

ZPML265 injection: 5 mg/kg, intravenous infusion (IV), D1, Q3W. The intravenous infusion lasted above 30 min. After completion of the infusion, the tube was flushed with 20 ml of the same solvent, with the entire infusion time (including the time of flushing the tube) of 60 min (±5 min). ZPML265 injection was not allowed to be administered by intravenous bolus.

### Chemotherapy regimen

If a subject's cumulative dosage of prior cisplatin treatment was ≤300 mg, the following regimen was used:
for a subject who was not treated by radical radiotherapy/adjuvant external pelvic irradiation, paclitaxel at 175 mg/m² on D1, cisplatin at 70 mg/m² on D2, with an administration frequency of Q3W;
for a subject who had received radical radiotherapy, paclitaxel at 135 mg/m² on D1, cisplatin at 50 mg/m² on D2, with an administration frequency of Q3W; and
for a subject who had received auxiliary external pelvic irradiation (≥50 Gy), paclitaxel at 135 mg/m² on D1, cisplatin at 60 mg/m² on D2, with an administration frequency of Q3W.

If a subject received a cumulative dosage of >300 mg of cisplatin prior to enrollment, the following regimen was used:
for a subject who was not treated by radical radiotherapy/adjuvant external pelvic irradiation, paclitaxel at 175 mg/m² on D1, carboplatin at AUC 6 on D1, with an administration frequency of Q3W;
for a subject who had received radical radiotherapy, paclitaxel at 135 mg/m² on D1, carboplatin at AUC 5 on D1, with an administration frequency of Q3W; and
for a subject who had received auxiliary external pelvic irradiation (≥50 Gy), paclitaxel at 135 mg/m² on D1, carboplatin at AUC 5-6 on D1, with an administration frequency of Q3W.

### Effectiveness Results

As of April 24, 2023, therapeutic efficacy data for evaluable cases were as follows. Objective remission rate (ORR) was 78.6% (22/28), disease control rate (DCR) was 100.0% (28/28), and median progression-free survival (mPFS) was 12.5 months. The therapeutic efficacy was superior to that of a similar species on the market, pembrolizumab (a PD-1 inhibitor).

### Safety Results

As of April 24, 2023, the safety data from 30 subjects were summarized. All subjects (100%) experienced treatment-emergent adverse events (TEAEs). Grade ≥3 treatment-related adverse events (TRAEs) occurred in 73.3% of the subjects (22 cases), most of which were chemotherapy-related adverse events. No treatment-related death events occurred, indicating good safety and tolerance.

### Preliminary Conclusions

ZPML265 injection in combination with paclitaxel-cisplatin/carboplatin showed a trend of significant therapeutic efficacy in patients with recurrent or metastatic cervical cancer with overall controllable safety and good tolerance.

### Example 14: A Phase II Clinical Study to Evaluate the Safety and Effectiveness of ZPML265 Injection in Combination with Paclitaxel-Cisplatin/Carboplatin and Bevacizumab for First-line Treatment of Patients with Recurrent or Metastatic Cervical Cancer

### Administration

In a phase II clinical study conducted in China, 30 patients with recurrent or stage IVB cervical cancer were enrolled, who were over 18 years old, had an Eastern Cooperative Oncology Group (ECOG) performance status score of 0 or 1, an expected survival of ≥ 3 months, and had the following functional levels of important organs prior to the first use of investigational product:
1) absolute neutrophil count ≥1.5×10⁹/L;
2) platelets≥100×10⁹/L;
3) hemoglobin ≥90 g/L;
4) serum albumin ≥30 g/L;
5) alanine aminotransferase (AST) and aspartate aminotransferase (ALT) ≤2.5× upper limit of normal (ULN) (≤5×ULN was permitted for patients with liver cancer or liver metastases);
6) total bilirubin ≤1.5×ULN (≤3×ULN was permitted for patients with Gilbert's syndrome);
7) serum creatinine ≤1.5×ULN, if the creatinine level of a patient was >1.5×ULN, creatinine clearance (CLcr) calculated by the Cockcroft-Gault equation must be≥50 mL/min;
8) left ventricular ejection fraction (LVEF)>50%;
9) proteinuria<2+(when urine protein of a patient was ≥2+, 24-hour urine protein should be quantified and the patient can be enrolled if the amount of the 24-hour urine protein was ≤1g).
10) international normalized ratio (INR) ≤1.5; and activated partial thromboplastin time (APTT) ≤1.5×ULN.

### Dosing Regimen

(1) ZPML265, 5 mg/kg, intravenous infusion, D1, Q3W;
(2) Paclitaxel, 175 mg/m² or 135 mg/m², intravenous infusion, D1, Q3W;
(3) Cisplatin, 50 mg/m², intravenous infusion, D1 or D2, Q3W;
(4) Carboplatin, AUC 5, intravenous infusion, D1, Q3W;
(5) Bevacizumab, 15 mg/kg, intravenous infusion, D1, Q3W.

The choice of cisplatin or carboplatin in the dosing regimen was at the discretion of the investigator based on subjects' conditions.

ZPML265 injection in combination with paclitaxel-cisplatin/carboplatin and bevacizumab was administered until disease progression, intolerable toxicity, initiation of new antitumor therapy, loss to follow-up, death, withdrawal from the study or other criteria for withdrawal from study treatment occurred (whichever occurred first).

At the discretion of the investigator, subjects should receive 6 cycles of chemotherapy, provided that they were tolerated. Subsequently, chemotherapy may be discontinued according to the investigator's decision and/or the subject's preference, and treatment with ZPML265 injection and bevacizumab was continued until disease progression or other criteria for withdrawal from study treatment occurred.

When a subject experienced disease progression but did not meet any criteria for withdrawal from study treatment, the subject was permitted to continue to receive ZPML265 monotherapy if the investigator deemed that the subject might still benefit from the continued treatment with ZPML265, until the investigator determined that there was no longer clinical benefit, or there was intolerable toxicity, initiation of new antitumor therapy, loss to follow-up, death, withdrawal from the study, or other criteria for discontinuation of study treatment (whichever occurred first).

Subjects in the study received up to 2 years (24 months) of study treatment.

Evaluation indicators. The endpoints of this study were safety and effectiveness indicators and other indicators, including:
1) Safety indicators;
2) Objective remission rate (ORR) (according to RECIST v1.1);
3) Duration of remission (DOR) (according to RECIST v1.1);
4) Disease control rate (DCR) (according to RECIST v1.1);
5) Progression-free survival (PFS) (according to RECIST v1.1);
6) Overall survival (OS);
7) Pharmacokinetic parameters of ZPML265; and
8) Immunogenicity of ZPML265.

### Effectiveness Results

As of April 24, 2023, therapeutic efficacy data for evaluable cases were as follows. Objective remission rate (ORR) was 83.3% (25/30), disease control rate (DCR) was 96.7% (29/30), and median progression-free survival (mPFS) was 16.4 months. The therapeutic efficacy was superior to that of a similar species on the market, pembrolizumab (a PD-1 inhibitor).

### Safety Results

As of April 24, 2023, the safety data from 30 subjects were summarized. All subjects (100%) experienced treatment-emergent adverse events (TEAEs). Grade ≥3 treatment-related adverse events (TRAEs) occurred in 70.0% of the subjects (21 cases), most of which were chemotherapy-related adverse events. No treatment-related death events occurred, indicating good safety and tolerance.

### Preliminary Conclusions

ZPML265 injection in combination with paclitaxel-cisplatin/carboplatin and bevacizumab showed a trend of significant therapeutic efficacy in patients with recurrent or metastatic cervical cancer with overall controllable safety and good tolerance.

The results of the above studies demonstrated that the formulation of ZPML265 injection in the present disclosure had excellent effects in terms of stability, safety, and therapeutic efficacy.

The embodiments of the present disclosure described above are exemplary only, and a person skilled in the art can recognize or ascertain innumerable equivalents to the specific compounds, materials, and procedures without the need for extraordinary experiments. All such equivalents are within the scope of this disclosure and are encompassed by the claims.

## Claims

1. An aqueous pharmaceutical composition comprising an antibody mixture of an anti-CTLA-4 antibody and an anti-PD-1 antibody, comprising:
1) the antibody mixture of the anti-CTLA-4 antibody and the anti-PD-1 antibody, wherein the heavy and light chains of the anti-CTLA-4 antibody have amino acid sequences set forth in SEQ ID NOs: 1 and 2, respectively, and the heavy and light chains of the anti-PD-1 antibody have amino acid sequences set forth in SEQ ID NOs: 3 and 4, respectively;
2) a stabilizer selected from the group consisting of sucrose and trehalose;
3) a surfactant selected from the group consisting of polysorbate 80, polysorbate 20, and poloxamer 188;
4) a buffer selected from the group consisting of acetic acid-sodium hydroxide, histidine-hydrochloric acid, and histidine-acetic acid;
wherein the aqueous pharmaceutical composition has a pH of about 4.0-6.0, and the molar ratio of the anti-CTLA-4 antibody to the anti-PD-1 antibody in the antibody mixture is about 1: 2.

2. The aqueous pharmaceutical composition of claim 1, wherein the antibody mixture is at a concentration of about 20-200 mg/ml.

3. The aqueous pharmaceutical composition of claim 1, wherein the stabilizer is in an amount of about 50-100 mg/ml.

4. The aqueous pharmaceutical composition of claim 1, wherein the surfactant is in an amount of about 0.1-2 mg/ml.

5. The aqueous pharmaceutical composition of claim 1, wherein the buffer is in an amount of about 5-50 mM.

6. The aqueous pharmaceutical composition of claims 1-5, comprising about 20-30 mg/ml of the antibody mixture, about 50-100 mg/ml of sucrose or about 50-100 mg/ml of trehalose, about 0.1-2 mg/ml of polysorbate 80 or about 0.1-2 mg/ml of polysorbate 20 or about 0.1-2 mg/ml of poloxamer 188, about 5-50 mM of acetic acid-sodium hydroxide or about 5-50 mM of histidine-hydrochloric acid or about 5-50 mM of histidine-acetic acid, wherein the aqueous pharmaceutical composition has a pH of about 4.0-6.0.

7. The aqueous pharmaceutical composition of claim 6, comprising about 20-30 mg/ml of the antibody mixture, about 80-100 mg/ml of sucrose or about 80-100 mg/ml of trehalose, about 0.1-0.5 mg/ml of polysorbate 80 or about 0.1-0.5 mg/ml of polysorbate 20 or about 0.1-0.5 mg/ml of poloxamer 188, about 10-50 mM of acetic acid-sodium hydroxide or about 10-50 mM of histidine-hydrochloric acid or about 10-50 mM of histidine-acetic acid, wherein the aqueous pharmaceutical composition has a pH of about 4.7-5.3.

8. The aqueous pharmaceutical composition of claim 1 or 2, further comprising an antioxidant and/or a dispersant.

9. The aqueous pharmaceutical composition of claim 8, wherein the antioxidant is methionine in an amount of about 0-50 mM and/or the dispersant is hyaluronidase in an amount of about 0-4000 IU/ml.

10. The aqueous pharmaceutical composition of claim 9, further comprising arginine hydrochloride in an amount of about 0-150 mM.

11. The aqueous pharmaceutical composition of claim 10, comprising about 100-200 mg/ml of the antibody mixture, about 50-100 mg/ml of sucrose or about 50-100 mg/ml of trehalose, about 0.1-2 mg/ml of polysorbate 80 or about 0.1-2 mg/ml of polysorbate 20 or about 0.1-2 mg/ml of poloxamer 188, about 5-50 mM of acetic acid-sodium hydroxide or about 5-50 mM of histidine-hydrochloric acid or about 5-50 mM of histidine-acetic acid, wherein the aqueous pharmaceutical composition has a pH of about 4.0-6.0, and optionally further comprising about 0-50 mM of methionine, and/or about 0-4000 IU/ml of hyaluronidase, and/or about 0-150 mM of arginine hydrochloride.

12. The aqueous pharmaceutical composition of claim 11, comprising about 100-200 mg/ml of the antibody mixture, about 50-100 mg/ml of sucrose or about 50-100 mg/ml of trehalose, about 0.1-2 mg/ml of polysorbate 80 or about 0.1-2 mg/ml of polysorbate 20 or about 0.1-2 mg/ml of poloxamer 188, about 5-50 mM of acetic acid-sodium hydroxide or about 5-50 mM of histidine-hydrochloric acid or about 5-50 mM of histidine-acetic acid, about 5-50 mM of methionine, about 500-4000 IU/ml of hyaluronidase, wherein the aqueous pharmaceutical composition has a pH of about 4.0-6.0, and optionally further comprising about 20-150 mM of arginine hydrochloride.

13. The aqueous pharmaceutical composition of claim 12, comprising about 100-200 mg/ml of the antibody mixture, about 60-90 mg/ml of sucrose or about 60-90 mg/ml of trehalose, about 0.2-1 mg/ml of polysorbate 80 or about 0.2-1 mg/ml of polysorbate 20 or about 0.2-1 mg/ml of poloxamer 188, about 10-20 mM of acetic acid-sodium hydroxide or about 10-20 mM of histidine-hydrochloric acid or about 10-20 mM of histidine-acetic acid, about 10 mM of methionine, about 2000 IU/ml of hyaluronidase, wherein the aqueous pharmaceutical composition has a pH of about 5.0-5.8, and optionally further comprising about 100 mM of arginine hydrochloride.

14. A lyophilized formulation, which is obtained by freeze-drying the aqueous pharmaceutical composition of any one of the preceding claims, or which, upon reconstitution, yields the aqueous pharmaceutical composition according to any one of the preceding claims.

15. Use of the pharmaceutical composition or lyophilized formulation of any one of the preceding claims in the manufacture of a medicament for the treatment of a tumor.

16. The use of claim 15, wherein the tumor is selected from the group consisting of esophageal squamous cell carcinoma, melanoma, cervical cancer, small cell lung cancer, non-small cell lung cancer, colorectal cancer, liver cancer, and renal cancer.
